# EUROPÄISCHE PATENTSCHRIFT

(11) **EP 0 628 312 B1**
(45) Veröffentlichungstag und Bekanntmachung des Hinweises auf die Patenterteilung: **26.04.2000**
(21) Anmeldenummer: 94250030.7
(22) Anmeldetag: 14.02.1994
(51) Int. Cl.: A61K 31/57, A61K 31/565

(54) **Kombinationspräparat zur Kontrazeption**
Anti-contraceptive composition
Composition anticonceptionnel

(30) Priorität: 12.03.1993 DE 4308406
(43) Veröffentlichungstag der Anmeldung: 14.12.1994
(73) Patentinhaber: Jenapharm GmbH & Co. KG, 07745 Jena (DE)
(72) Erfinder: Oettel, Michael, Prof., D-07743 Jena (DE); Dittgen, Michael, Prof., D-99510 Apolda (DE); Osterwald, Hermann, Dr., D-07743 Jena (DE)
(74) Vertreter: Wablat, Wolfgang, Dr.Dr.

(56) Entgegenhaltungen:
- US-A- 4 372 951

## Beschreibung

Die Erfindung betrifft pharmazeutische Kombinationspräparate zur Kontrazeption unter oraler, intravaginaler und transdermaler Applikation.

Orale Kontrazeptiva kamen erstmals in den frühen 60er Jahren auf den Markt. Durch fortlaufende Forschungsarbeiten gelang es, die notwendigen Dosierungen an Hormonen schrittweise zu reduzieren. Heute existieren niedrig dosierte orale Kontrazeptiva, die hauptsächlich aus einer Estrogenkomponente und einer Gestagenkomponente bestehen. Dabei werden die Hormongaben in unterschiedlichsten Kombinationen und Dosierungen in Form von Stufen- oder Phasenpräparaten über einen Zeitraum von 21 bzw. 28 Tagen verabreicht.

Für die Gestagenkomponente werden als Wirkstoffe in ihrer chemischen Struktur sehr unterschiedliche Steroide verwendet.

Als Estrogenkomponente wird bevorzugt das bereits seit über 40 Jahren bekannte Ethinylestradiol oder dessen 3-Methylester, das Mestranol, verwendet. Mestranol ist ein Prodrug und wird im Körper zu Ethinylestradiol metabolisiert.

Ethinylestradiol weist allerdings eine Reihe von Nachteilen und Nebenwirkungen auf. Ethinylestradiol wird nur schlecht und individuell äußerst unterschiedlich im Magen-Darm-Trakt resorbiert. Dies führt zu einer nur ungenügenden Bioverfügbarkeit. Ferner wirkt Ethinylestradiol als suizidaler Hemmer auf das Cytochrom P₄₅₀-System.

Dadurch kommt es zu einer Hemmung der eigenen Abbau- und Metabolisierungswege. Da Gestagene und auch viele andere Wirkstoffe zum großen Teil über die gleichen Abbau- und Metabolisierungswege im Stoffwechsel laufen, führt dies ferner nach wiederholter Applikation zu einer Kumulation der Wirkstoffe im Körper (W. Carol, G. Klinger, W. Michels, J. Boer, Ch. Pocha: Untersuchungen zur Pharmakokinetik kontrazeptiver Steroide unter den Bedingungen der Langzeitaufnahmen; Zent.-Bl. Gynäkol. 113; S. 1298-1303; 1991). Dennoch konnten bisher keine alternativen Wirkstoffe zu Ethinylestradiol bzw. Mestranol für das Gebiet der oralen Kontrazeptiva gefunden werden.

So wird beispielsweise in der DE-PS 32 29 612 beschrieben, daß eine tägliche Dosierung von 0,020 mg bis 0,040 mg Ethinylestradiol neben 0,3 mg bis 0,8 mg Norethindron als Gestagen zur sicheren Kontrazeption erforderlich ist.

Die wesentlich verträglicheren körpereigenen (biogenen) Estrogene, wie 17β-Estradiol oder Estron, müssen entweder in mikronisierter oder in konjugierter Form, beispielsweise als Estradiolvalerat oder Estronsulfat, oral oder auch transdermal appliziert werden. Allerdings werden erst in sehr hohen Dosierungen von mehr als 4 mg/Tag die erforderlichen Blutspiegel erreicht, die eine ausreichende Proliferation des Endometriums und somit eine gute Zyklusstabilität während der Einnahme garantieren (R. A. Lobo und D. L. Cassidenti: Pharmacokinetics of oral 17β-Estradiol; J. Reprod. Med 37; S. 77-84; 1992). Aus diesem Grunde werden körpereigene Estrogene bisher nicht im größeren Maße zur Kontrazeption verwendet.

In US-PS 4 372 951 wird ein pharmazeutisches System beschrieben, das während der Follikelreifungsphase ein Estrogen-aktives Steroid oder ein Progesteron-aktives Steroid, und während der Gelbkörperphase eine Kombination von Estrogen und Progesteron verabreicht. Es wird vorgeschlagen, daß neben natürlichen Estrogen und natürlichem Progesteron auch synthetische Estrogene zum Einsatz kommen können.

Bei der Verwendung von Estradiol, einem biogenen Estrogen, sind nach der DE-PS 41 04 385 Dosierungen von 4 mg/Tag neben 1 mg/Tag Norethisteronacetat als Gestagen zur Erzielung einer zuverlässigen Kontrazeption erforderlich.

In mehreren US-Patenten (US-PS 4 530 839, 4 544 554, 4 628 051 und 4 921 843) werden verschiedene mehrphasige orale Kontrazeptiva beschrieben, gemäß denen über den gesamten Zyklus eine konstante Menge an Ethinylestradiol und beispielsweise ab dem 9. Tag des Zyklus eine konstante Menge bzw. ansteigende Mengen eines Gestagens zur Verbesserung der Zyklusstabilität verabreicht werden.

Aufgabe der vorliegenden Erfindung ist es, eine neue Wirkstoffkombination in mehreren Stufen zu schaffen, bei der die Gesamtmengen an zu verabreichenden Hormonen deutlich verringert sind.

Eine weitere Aufgabe der vorliegenden Erfindung ist es, Arzneimittel in Form von Tagesdosiseinheiten zu schaffen, die zur oralen, intravaginalen und transdermalen Applikation der neuen Wirkstoffkombination dienen.

Aufgabe der vorliegenden Erfindung ist ferner die Schaffung einer pharmazeutischen Packung, welche die erfindungsgemäßen Arzneimittel in der erforderlichen Anzahl und Reihenfolge von Tagesdosiseinheiten der einzelnen Stufen zur Verfügung stellt.

Die Aufgabe wird erfindungsgemäß dadurch gelöst, daß Ethinylestradiol oder dieses Estrogen im Körper abspaltende Verbindungen, gleichzeitig zusammen mit körpereigenen biogenen Estrogenen oder diese Estrogene im Körper abspaltende Verbindungen, verabfolgt wird.

Das erfindungsgemäße Kombinationspräparat zur Kontrazeption besteht aus einer Stufe oder aus einer und mehreren weiteren Stufen, wobei mindestens eine Stufe die Kombination von drei Komponenten, nämlich einem biogenen Estrogen, einem synthetischen Estrogen und einem Gestagen, enthält, und wobei die weiteren Stufen, jeweils aus einem pharmazeutisch unbedenklichen Placebo oder einem biogenen oder synthetischen Gestagen oder einem biogenen oder synthetischen Estrogen oder aus einer Kombination aus drei Komponenten, nämlich einem biogenen Estrogen, einem synthetischen Estrogen und einem Gestagen oder einer Kombination aus synthetischen Estrogenen und einem Gestagen bestehen.

Das biogene Estrogen weist erfindungsgemäß mindestens einen Bestandteil aus der Gruppe Estradiol, Estron, Estran, Estriol oder mindestens eine Verbindung, die einen der vorgenannten Estrogenbestandteile nach Einnahme schnell abspaltet, auf.

Das synthetische Estrogen weist erfindungsgemäß mindestens einen Bestandteil aus der Gruppe Ethinylestradiol, Mestranol oder mindestens eine Verbindung, die einen der vorgenannten Estrogenbestandteile nach Einnahme schnell abspaltet, auf.

Das Gestagen weist erfindungsgemäß mindestens einen Bestandteil aus der Gruppe Levonorgestrel, Desogestrel, Progesteron, Norethisteronacetat, Chlormadinonacetat, Gestoden, Cyproteronacetat oder mindestens eine Verbindung, die einen der vorgenannten Gestagenbestandteile nach Einnahme schnell abspaltet, auf.

Überraschenderweise wurde gefunden, daß die gleichzeitige Verabfolgung von synthetischen und biogenen Estrogenen zu einem synergistischen Effekt führt. Die Dosierungen der synthetischen und der biogenen Estrogene sind im einzelnen und insgesamt geringer als die zur Erzielung des gleichen Effektes notwendigen Dosierungen der jeweiligen Einzelkomponenten. Die zusätzlich zu verabfolgende Dosis an Gestagenen liegt dabei im Bereich vergleichbarer Präparate des Standes der Technik.

Eine kontrazeptiv wirksame erfindungsgemäße Kombination enthält
a) 0,005 bis 0,010 mg/Tag Ethinylestradiol oder
   Mestranol und
b) 1 mg/Tag Estradiol oder
   Estradiolvalerat oder
   Estronsulfat und
c) 0,125 mg/Tag Levonorgestrel oder
   0,150 mg/Tag Desogestrel oder
   0,6 mg/Tag Norethisteronacetat oder
   2 mg/Tag Dienogest oder
   2 mg/Tag Chlormadinonacetat.

Die Applikation erfolgt über einen Zeitraum der ersten 21 Tage des Zyklus und hat eine sichere kontrazeptive Wirkung. An den übrigen Tagen des Zyklus wird pharmazeutisch unbedenkliches Placebo oder überhaupt kein pharmazeutisches Präparat appliziert.

Durch die gleichzeitige Applikation von synthetischen und körpereigenen biogenen Estrogenen kann die Gesamtwirkstoffmenge erheblich reduziert werden. Dies führt zu einer Verminderung der Nebenwirkungen, die sonst durch die hohen Dosierungen an synthetischen oder körpereigenen Estrogenen hervorgerufen werden.

Eine andere Zusammensetzung und stufenmäßige Abfolge der erfindungsgemäßen Wirkstoffkombination sieht für die ersten 21 Tage des Zyklus die Applizierung der gleichen Wirkstoffe in der gleichen Dosierung vor, nur werden vom 22. bis zum 28. Tag des Zyklus körpereigene Estrogene appliziert, wie beispielsweise 2 mg/Tag Estradiolvalerat oder 1,5 mg/Tag Estronsulfat.

Eine weitere stufenförmige Abfolge der erfindungsgemäßen Wirkstoffkombination ist die Applikation von 0,01 mg/Tag Ethinylestradiol bzw. eine äquivalente Menge an Mestranol in Kombination mit 1,0 mg/Tag Estradiolvalerat oder Estronsulfat vom 1. bis zum 6. Zyklustag. Vom 7. bis zum 21. Tag werden 0,005 mg/Tag Ethinylestradiol zusammen mit 1 mg/Tag Norethisteronacetat bzw. 3 mg/Tag Dienogest oder Chlormadinonacetat oder einer äquivalenten Menge einen anderen Gestagens verabreicht. An den übrigen Tagen des Zyklus wird pharmazeutisch unbedenkliches Placebo oder überhaupt kein pharmazeutisches Präparat appliziert.

Eine weitere Anwendung der erfindungsgemäßen Wirkstoffkombination in Form eines mehrstufigen Kombinationspräparates ist die Applikation von 0,005 mg/Tag Ethinylestradiol oder einer äquivalenten Menge Mestranol zusammen mit 1,5 mg/Tag der körpereigenen Estrogene Estradiolvalerat oder Estronsulfat zusammen mit 0,05 mg/Tag Levonorgestrel oder 0,8 mg/Tag Dienogest oder einer äquivalenten Menge eines anderen Gestagens vom 1. bis zum 6. Zyklustag. Vom 7. bis zum 12. Zyklustag wird eine Kombination aus 0,0075 mg/Tag Ethinylestradiol und 2 mg/Tag Estradiolvalerat oder Estronsulfat und 0,75 mg/Tag Levonorgestrel oder 12 mg/Tag Dienogest oder Chlormadinonacetat oder der äquivalenten Menge eines anderen Gestagens verabfolgt. Vom 13. bis zum 21. Zyklustag werden 0,005 mg/Tag Ethinylestradiol und 1,5 mg/Tag Estradiolvalerat und 0,125 mg/Tag Levonorgestrel oder 2 mg/Tag Dienogest oder eine äquivalente Menge eines anderen Gestagens appliziert. An den übrigen Tagen des Zyklus wird pharmazeutisch unbedenkliches Placebo oder überhaupt kein pharmazeutisches Präparat appliziert.

Die erfindungsgemäßen Wirkstoffkombinationen werden in Form von oral anwendbaren galenischen Zubereitungsformen appliziert. Als orale Zubereitungsformen kommen beispielsweise Tabletten, Dragees, Pillen oder Kapseln in Frage. Die Zubereitungsformen werden in an sich üblicher Weise unter Verwendung üblicher Hilfs- und Trägerstoffe hergestellt, wie sie beispielsweise im "Remington's Pharmaceutical Sciences Handbook, Hack Pub. Co., N.Y., USA" beschrieben sind.

Eine Variante der Applikation der erfindungsgemäßen Wirkstoffkombination stellt die Applikation in Form von Vaginalzäpfchen und Vaginalkapseln dar. Dazu werden Vaginalzäpfchen und Vaginalkapseln nach den üblichen Methoden unter Verwendung der üblichen Hilfsstoffe hergestellt.

Die erfindungsgemäßen Wirkstoffkombinationen können ferner in Form von transdermalen therapeutischen Systemen (TTS) appliziert werden. Dazu werden die erfindungsgemaßen Wirkstoffkombinationen in an sich bekannter Weise in ein TTS eingebracht. Das TTS kann beispielsweise auf Iontophorese oder Diffusion oder gegebenenfalls auf Kombinationen dieser Effekte beruhen.

Das TTS wird an geeigneter Stelle am Körper angebracht. Die Wirkstoffe werden dann transcutan appliziert, wobei die Applikationsrate durch die Größe der Fläche des TTS und durch die gegebenenfalls angelegte Spannung gesteuert wird.

Für die bevorzugte orale Applikation werden die erfindungsgemäßen Kombinationspräparate zur Kontrazeption zweckmäßig in Form einer pharmazeutischen Packung zusammengefaßt, die die tägliche Dosierung darstellende Formulierung in fortlaufender Reihenfolge unterbringt.

Gegenstand der vorliegenden Erfindung sind somit auch pharmazeutische Packungen, die dadurch gekennzeichnet sind, daß sie Dosierungseinheiten in abgestimmter, festgelegter Reihenfolge enthalten, wobei die Reihenfolge den Stufen der täglichen Verabfolgung entspricht.

Die pharmazeutische Packung kann beispielsweise in Form einer Tiefziehpackung hergestellt werden. Darin werden zum Beispiel 6 Dragees der ersten Stufe (Kombination von Gestagenen und biogenen und synthetischen Estrogenen), 6 Dragees der zweiten Stufe (Kombination wie bei der ersten Stufe), 9 Dragees der dritten Stufe (Kombination wie bei der ersten Stufe) und 7 Placebos verpackt, die jeweils täglich, also über 28 Tage entnommen werden können. Eine Packung für 21 wird ohne die 7 Placebos ausgeführt. Abwandlungen bezüglich der täglichen Dosierung, der Ausführungen der Applikationsformen, der Form der Packung etc. sind dem Fachmann geläufig.

### Beispiel 1

Es wurde ein ovulationshemmendes Mittel verwendet, welches 21 Tageseinheiten mit jeweils 0,01 mg Ethinylestradiol, 1,0 mg Estradiolvalerat und 0,125 mg Levonorgestrel enthielt. An den Zyklustagen 22 bis 28 wurde kein Präparat eingenommen. Das Mittel wurde ein Jahr lang verwendet und zeigte bei sehr guter kontrazeptiver Sicherheit praktisch keine Nebenwirkungen, wobei Zwischenblutungen deutlich seltener auftraten als bei einer herkömmlichen Kombination von täglich 0,03 mg Ethinylestradiol und 0,125 mg Levonorgestrel. Es ergaben sich keine Anzeichen für Stoffwechsel- oder Herz-Kreislauf-Nebenwirkungen. Im Gegensatz zu herkömmlichen Kontrazeptiva mit höherer Ethinylestradiol-Dosierung war die Koffein-Elimination als möglicher Ausdruck einer negativen Beeinträchtigung arzneimittelabbauender Systeme nicht beeinflußt.

### Beispiel 2

Zur ovulationshemmenden Behandlung wurde ein zweistufiges Sequenzpräparat verwendet, welches 21 Tageseinheiten mit jeweils 0,005 mg Ethinylestradiol, 2 mg mikronisiertes 17β-Estradiol und 2 mg Dienogest und 7 Tageseinheiten mit jeweils 2 mg mikronisiertem 17β-Estradiol enthielt. Das Mittel wurde ein Jahr lang verabreicht. Bei hoher kontrazeptiver Sicherheit war die Zykluskontrolle hervorragend, d. h. es wurden im Vergleich zu herkömmlichen Kontrazeptiva keine Zwischenblutungen registriert. Die Dauer und Intensität der regelmäßigen Abbruchblutungen entsprachen den Befunden bei herkömmlichen oralen Kontrazeptiva.
Im Gegensatz zu herkömmlichen Kontrazeptiva mit höherer Ethinylestradiol-Dosierung war die Verstoffwechselung und die Ausscheidung von Nifedipin als Ausdruck einer negativen Beeinträchtigung arzneimittelabbauender Enzyme nicht beeinflußt.

### Beispiel 3

Zur hormonalen Kontrazeption wurde ein ovulationshemmendes Mittel in Form eines zweistufigen Präparates verwendet. Zunächst wurde über 6 Tage eine Kombination von täglich 1,5 mg Estronsulfat und 0,01 mg Mestranol und anschließend an den Zyklustagen 7 bis 21 täglich 1,0 mg Estronsulfat, 0,01 mg Mestranol und 1,0 mg Norethisteronacetat verabfolgt. An den Zyklustagen 22 bis 28 bestand eine Einnahmepause. Das Mittel wurde 8 Monate lang verabreicht. Die kontrazeptive Sicherheit war zufriedenstellend, die Akzeptanz sehr gut. Die Frauen im Alter von 30 bis 38 Jahren berichteten über stimmungsaufhellende Effekte.

### Beispiel 4

Es wurde zur oralen Kontrazeption ein ovulationshemmendes Mittel in Form eines dreistufigen Präparates verwendet, welches 6 Tageseinheiten mit jeweils 0,005 mg Ethinylestradiol, 1,0 mg Estradiolvalerat und 0,075 mg Desogestrel, weitere 6 Tageseinheiten mit jeweils 0,005 mg Ethinylestradiol, 1,5 mg Estradiolvalerat und 0,100 mg Desogestrel und schließlich 9 Tageseinheiten mit 0,005 mg Ethinylestradiol, 1,0 mg Estradiolvalerat und 0,150 mg Desogestrel enthielt. An den Zyklustagen 22 bis 28 bestand eine Einnahmepause. Das Medikament wurde 11 Monate lang verabreicht. Die kontrazeptive Sicherheit war sehr gut, die Zwischenblutungsrate relativ niedrig. Es gab keine Probandin, welche die Medikation abgebrochen hatte.

### Beispiel 5

Zur ovulationshemmenden Behandlung wurde ein vierstufiges Kontrazeptivum eingesetzt, das aus 6 Tageseinheiten mit jeweils 0,01 mg Ethinylestradiol, 1,0 mg Estradiolvalerat und 0,03 mg Levonorgestrel, 6 Tageseinheiten mit jeweils 0,01 mg Ethinylestradiol, 1,0 mg Estradiolvalerat und 0,05 mg Levonorgestrel, 9 Tageseinheiten mit jeweils 0,01 mg Ethinylestradiol, 1,0 mg Estradiolvalerat und 0,100 mg Levonorgestrel und schließlich 7 Tageseinheiten mit jeweils 2,0 mg Estradiolvalerat bestand. Die Behandlungsdauer betrug 12 Monate. Die kontrazeptive Sicherheit entsprach derjenigen von Beispiel 4, jedoch war die Zykluskontrolle deutlich besser.

### Beispiel 6

Zur kontrazeptiven Medikation wurde folgende Kombinations-Behandlung angewendet: 21 Tageseinheiten mit jeweils 0,015 mg Mestranol und 0,05 mg Gestoden. Zusätzlich wurde in der Zeit zwischen den Tagen 1 und 28 zweimal wöchentlich je ein transdermales therapeutisches System mit jeweils 4 mg 17β-Estradiol auf die äußere Haut der oberen Gesäßhälfte aufgeklebt. Die Studie umfaßte insgesamt 500 Zyklen. Die kontrazeptive Sicherheit entsprach derjenigen konventioneller Kontrazeptiva. Es gab keinen Fall von prämenstruellen Beschwerden (Unwohlsein, Schwindelgefühl, Hitzewallungen usw.).

### Beispiel 7

Bei jüngeren Frauen mit Kohabitationsbeschwerden wurde folgendes kontrazeptives Schema ein Jahr lang angewendet: 21 Tageseinheiten mit jeweils 0,005 mg Ethinylestradiol und 1,5 mg Dienogest. Zusätzlich wurde an den Tagen 1 bis 28 alle zwei Tage eine sich schnell auflösende Vaginalkapsel mit jeweils 10 mg Estriol eingesetzt. Die kontrazeptive Sicherheit lag in der Größenordnung herkömmlicher Kontrazeptiva. Das Scheidenepithel war im Vergleich zum Ausgangszustand gut durchblutet. Die erhöhte vaginale Lubrifikation wurde von den Patientinnen als sehr angenehm empfunden, so daß die Rate der Kohabitationsbeschwerden deutlich zurückging.

## Patentansprüche

1. Kombinationspräparat zur Kontrazeption, bestehend aus einer Stufe oder aus einer und mehreren weiteren Stufen, wobei mindestens eine Stufe die Kombination von drei Komponenten, nämlich einem biogenen Estrogen, einem synthetischen Estrogen und einem Gestagen enthält, und wobei die weiteren Stufen jeweils aus einem pharmazeutisch unbedenklichen Placebo bestehen
oder ein biogenes oder synthetisches Gestagen,
oder ein biogenes oder synthetisches Estrogen,
oder eine Kombination aus drei Komponenten, nämlich ein biogenes Estrogen, ein synthetisches Estrogen und ein Gestagen
oder eine Kombination aus synthetischen Estrogenen und einem Gestagen enthalten.

2. Kombinationspräparat nach Anspruch 1, dadurch gekennzeichnet, daß das biogene Estrogen mindestens einen Bestandteil aus der Gruppe Estradiol, Estron, Estran, Estriol oder mindestens eine Verbindung, die einen der vorgenannten Estrogenbestandteile nach Einnahme schnell abspaltet, aufweist.

3. Kombinationspräparat nach Anspruch 1, dadurch gekennzeichnet, daß das synthetische Estrogen mindestens einen Bestandteil aus der Gruppe Ethinylestradiol, Mestranol oder mindestens eine Verbindung, die einen der vorgenannten Estrogenbestandteile nach Einnahme schnell abspaltet, aufweist.

4. Kombinationspräparat nach Anspruch 1, dadurch gekennzeichnet, daß das Gestagen mindestens einen Bestandteil aus der Gruppe Levonorgestrel, Desogestrel, Progesteron, Norethisteronacetat, Chlormadinonacetat, Gestoden, Cyproteronacetat oder mindestens eine Verbindung, die einen der vorgenannten Gestagenbestandteile nach Einnahme schnell abspaltet, aufweist.

5. Kombinationspräparat nach den Ansprüchen 1 bis 4 zur oralen Anwendung.

6. Kombinationspräparat nach den Ansprüchen 1 bis 4 zur intravaginalen Anwendung.

7. Kombinationspräparat nach den Ansprüchen 1 bis 4 zur transdermalen Anwendung.

8. Pharmazeutische Packung zur Kontrazeption, dadurch gekennzeichnet, daß sie bis zu 28 Tagesdosiseinheiten des Kombinationspräparates nach mindestens einem der vorangehenden Ansprüche enthält, wobei die Anzahl der Tagesdosiseinheiten der ersten Stufe 1 bis 28 beträgt und im Falle des Vorliegens einer zweiten Stufe die Anzahl der Tagesdosiseinheiten für die zweite Stufe 1 bis 14 beträgt und im Falle des Vorliegens weiterer Stufen die Anzahl der Tagesdosiseinheiten für diese weiteren Stufen jeweils 1 bis 7 beträgt,
wobei mindestens eine Stufe die Kombination von drei Komponenten, nämlich einem biogenen Estrogen, einem synthetischen Estrogen und einem Gestagen enthält, und wobei die weiteren Stufen jeweils aus einem pharmazeutisch unbedenklichen Placebo bestehen
oder ein biogenes oder synthetisches Gestagen,
oder ein biogenes oder synthetisches Estrogen,
oder eine Kombination aus drei Komponenten, nämlich ein biogenes Estrogen, ein synthetisches Estrogen und ein Gestagen
oder eine Kombination aus synthetischen Estrogenen und einem Gestagen enthalten.

## Claims

1. A combination preparation for contraception, consisting of one step or of one and several additional steps, at least one step containing a combination of three components, namely a biogenic estrogen, a synthetic estrogen and a gestagen, and each of the other steps consisting of a pharmaceutically unobjectionable placebo
or a biogenic or synthetic gestagen,
or a biogenic or synthetic estrogen,
or a combination of three components, namely a biogenic estrogen, a synthetic estrogen, and a gestagen
or a combibation of synthetic estrogens and a gestagen.

2. A combination preparation according to Claim 1, characterized by the fact that the biogenic estrogen has at least one component selected from the group consisting of estradiol, estrone, estrane, estriol, or at least one compound which, when administered, rapidly splits off one of the aforementioned estrogen components.

3. A combination preparation according to Claim 1, characterized by the fact that the synthetic estrogen has at least one component selected from the group consisting of ethinyl estradiol, mestranol, or at least one compound which, when administered, rapidly splits off one of the aforementioned estrogen components.

4. A combination preparation according to Claim 1, characterized by the fact that the gestagen has at least one component selected from the group consisting of levonorgestrel, desogestrel, progesterone, norethisterone acetate, chlormadinone acetate, gestoden, cyproterone acetate, or at least one compound which, when administered, rapidly splits off one of the aforementioned gestagen components.

5. A combination preparation according to Claims 1 to 4 for oral administration.

6. A combination preparation according to Claims 1 to 4 for intravaginal administration.

7. A combination preparation according to Claims 1 to 4 for transdermal administration.

8. A pharmaceutical package for contraception, characterized by the fact that it contains up to 28 daily dose units of the combination preparation according to at least one of the preceding claims, the number of daily dose units of the first step being 1 to 28 and, in case of the presence of a second step, the number of the daily dose units for the second step being 1 to 14 and, in the case of the presence of further steps, the number of daily dose units for such further steps being in each case 1 to 7,
whereby at least one step containing a combination of three components, namely a biogenic estrogen, a synthetic estrogen and a gestagen, and each of the other steps consisting of a pharmaceutically unobjectionable placebo
or a biogenic or synthetic gestagen,
or a biogenic or synthetic estrogen,
or a combination of three components, namely a biogenic estrogen, a synthetic estrogen, and a gestagen
or a combibation of synthetic estrogens and a gestagen.

## Revendications

1. Préparation combinée pour la contraception, constituée d'un seul étage ou d'un et plusieurs étages additionnels, dans laquelle au moins un étage contient la combinaison de trois composants, notamment un oestrogène biogène, un oestrogène synthétique et un gestagène, et dans laquelle les étages additionnelles consistent le cas échéant en un placebo pharmaceutique acceptable, ou contiennent
un gestagène biogène synthétique,
un oestrogène biogène synthétique,
ou une combinaison de trois composants, notamment un oestrogène biogène, un oestrogène synthétique et un gestagène,
ou une combinaison d'oestrogènes synthétiques et d'un gestagène.

2. Préparation combinée selon la revendication 1, caractérisée en ce que l'oestrogène biogène comprend au moins un composant du groupe de l'oestradiol, de l'oestrone, l'oestrane, l'oestriol ou au moins un composé qui, après administration, libère rapidement un des composants oestrogènes précités.

3. Préparation combinée selon la revendication 1, caractérisée en ce que l'oestrogène synthétique comprend au moins un composant choisi dans le groupe de l'ethinyloestradiol, du mestranol ou au moins un composé qui, après administration, libère rapidement un des composants oestrogènes précités.

4. Préparation combinée selon la revendication 1, caractérisée en ce que le gestagène comporte au moins un composant choisi dans le groupe du levonorgestrel, du desogestrel, de la progestérone, du norethisteronacétate, du chlormadinonacétate, du gestodène, du cyproteronacétate ou au moins un composé qui, après administration, libère rapidement un des composés précités.

5. Préparation combinée selon l'une des revendications 1 à 4 pour administration orale.

6. Préparation combinée selon l'une des revendications 1 à 4 pour administration intra vaginale.

7. Préparation combinée selon l'une des revendications 1 à 4 pour administration transdermique.

8. Kit pharmaceutique pour la contraception caractérisé en ce qu'il comprend jusqu'à 28 doses unitaires journalières de la préparation combinée selon l'une quelconque des revendications précédentes, la quantité des doses unitaires journalières du premier étage représentant de 1 à 28 et dans le cas de la présence d'un deuxième étage, le nombre des doses unitaires journalières du deuxième étage représentant de 1 à 14 et dans le cas de la présence d'étages additionnels, le nombre de doses unitaires journalières de ces étages additionnels représentant de 1 à 7,
dans lequel au moins un étage comprend la combinaison de trois composants, notamment un oestrogène biogène, un oestrogène synthétique et un gestagène, et dans lequel les étages additionnels comprennent chacun un placebo pharmaceutiquement acceptable ou un gestagène biogène ou synthétique, ou un oestrogène biogène ou synthétique, ou une combinaison de trois composants, notamment un oestrogène biogène, un oestrogène synthétique et un gestagène,
ou une combinaison d'oestrogènes synthétiques et d'un gestagène.
